# EUROPÄISCHE PATENTANMELDUNG

(11) **EP 1 391 737 A2**
(43) Veröffentlichungstag der Anmeldung: **25.02.2004**
(21) Anmeldenummer: 03016763.9
(22) Anmeldetag: 23.07.2003
(51) Int. Cl.: G01R 1/067

(54) **Vorrichtung zur gleichzeitigen Einspeisung von elektrischen Signalen und zur Messung des Potentials in Proben**

(30) Priorität: 23.08.2002 DE 10238823
(71) Anmelder: FORSCHUNGSZENTRUM JÜLICH GMBH, 52425 Jülich (DE)
(72) Erfinder: Tillmann, Axel, Dr., 52457 Aldenhoven (DE); Kemna, Andreas, Dr., 40221 Düsseldorf (DE); Zimmermann, Egon, 52459 Inden-Altdorf (DE); Glaas, Walter, 50189 Elsdorf (DE); Verweerd, Arre, 52070 Aachen (DE)

(57) **Zusammenfassung**

Die Erfindung betrifft eine Vorrichtung zur Einspeisung von Wechselströmen und gleichzeitigen Potential-Messung. Die Vorrichtung ist durch zwei galvanisch entkoppelten Elektrodenflächen in einem Elektrodenspieß (100, 101) gekennzeichnet.

Dabei dient als Potentialelektrode die Spitze des Elektrodenspießes. Die Spitze dient somit der Ankopplung an die Probe zwecks Potentialmessung. Der Mantel des Elektrodenspießes dient als Elektrode zur Einspeisung des Wechselstroms. Je nach Eindringtiefe des Elektrodenspießes in die Probe existiert somit eine variable Ankopplungsfläche zur Einspeisung des Wechselstroms in die Probe.
Vorteilhaft ist die Vorrichtung einfach aufgebaut. Meßfehler durch Polarisationseffekte sind ausgeschlossen. Zur Installation solcher Elektrodenspieße benötigt man gegenüber der Installation von Elektroden aus dem Stand der Technik nur noch die Hälfte der Zeit. Die Untersuchung einer gegebenen Probenfläche bzw. Probeninhalts wird dadurch beschleunigt, insbesondere bei der Untersuchung heterogener Proben, wie Böden, bei denen mit vielen Elektroden gleichzeitig gearbeitet werden muss.

## Beschreibung

Die Erfindung betrifft eine Vorrichtung zur gleichzeitigen Einspeisung von elektrischen Signalen und zur Messung des Potentials in Proben.

Die Messung von Potentialdifferenzen in einer zu untersuchenden Probe ist eine Voraussetzung unter anderem zur Berechnung der elektrischen Leitfähigkeit der Probe. Hierzu werden die zu untersuchenden Proben in der Regel mit Strömen angeregt. Aus dem gemessenen Spannungsabfall und der in der Zuleitung gemessenen Stromstärke wird der elektrische Widerstand und als Kehrwert hieraus die Leitfähigkeit der Probe berechnet.

Aus dem Stand der Technik sind verschiedene Meßanordnungen und Verfahren zur Messung der elektrischen Leitfähigkeit bekannt.
Grundsätzlich umfassen diese mindestens ein Elektrodenpaar. Die sogenannte Kohlrausch-Anordnung umfaßt gewöhnlich zwei sich gegenüber liegende Festkörper-Elektroden, beispielsweise aus Kohle oder platiniertem Platin, deren Gestalt unterschiedlich sein kann.
Zur Durchführung der elektrischen Leitfähigkeitsmessung wird eine Wechselspannung angelegt und Ströme in eine elektrisch leitfähige Probe z. B. durch eine in die Probe eingeführte Elektrode eingespeist. Die dabei erzeugten elektrischen Potentiale werden ebenfalls an solchen, mit den zur Stromeinspeisung baugleichen, Elektroden gemessen.

Eine einfache Art der Durchführung solcher Messungen sind sogenannte "Pol-Pol-Messungen", die mit zwei Elektroden durchgeführt werden (Zwei-Elektroden-Anordnung). Dabei werden sowohl der Strom als auch die Potentialdifferenz zwischen den beiden Elektroden gemessen.
Nachteilig werden die Meßergebnisse in erheblichem Maße durch Polarisationseffekte an der Grenzfläche zwischen den Elektroden und der Probe verfälscht. Im besonderen Falle von Böden oder anderen heterogen strukturierten Proben treten zwischen der Probe und den stromeinspeisenden Elektroden vom Einspeisungspunkt abhängige hohe Übergangswiderstände auf.

Als Ausweg hieraus können Potentialmessungen mit der Vier-Punkt-Methode (Vier-Elektroden-Anordnung) durchgeführt werden.
Dabei werden zwei Elektroden zur Einspeisung von Strömen und zwei weitere zur Potentialdifferenzmessung in der Probe installiert. Die Elektroden dienen zu einem gegebenen Zeitpunkt entweder zur Einspeisung des Wechselstroms oder zur hochohmigen Messung des Potentials. Aus der bekannten Stromstärke und der gemessenen Potentialdifferenz wird der Widerstand bzw. die Leitfähigkeit der Probe berechnet. Die Übergangswiderstände und zusätzliche Potentialdifferenzen durch Polarisationseffekte am Übergang Probe zu Elektrode werden bei dieser Messung nicht erfaßt.

Nachteilig müssen dann aber zur gleichzeitigen Messung von Potentialdifferenz und Stromstärke an einer Stelle der Probe zwei dicht beieinander angeordnete Elektroden in die Probe eingebracht werden. Alternativ können aufwendig konstruierte und daher teure Elektroden eingebracht werden. Bei heterogen strukturierten und dichten Proben wie beispielsweise Böden, Aquiferen, Gestein, Erzlagerstätten usw. müssen eine Vielzahl an Elektroden in diese Proben eingebracht werden. Bei flächenhaften und / oder tomographischen Messungen solcher Proben dienen die Elektroden aufgrund der großen Anzahl von verschiedenen Einspeisungs- und Meßkonfigurationen abwechselnd als Einspeisungs- oder Meßelektrode. Die Meßanordnung kann dabei für solch komplexe Proben, insbesondere innerhalb der Geophysik und der Geoelektrik, aufwendig gestaltet sein, wie aus der Druckschrift DE 198 37 828 bekannt ist.

Je nach Durchführungsmethode, ergeben sich somit unterschiedliche Nachteile. Prinzipbedingt treten bei allen Messungen, die an einer Elektrode gleichzeitig Strom einspeisen und das Potential messen, wie z. B. bei der Zwei-Elektroden-Meßanordnung, große Meßfehler aufgrund der hohen, variablen und unbekannten Übergangswiderstände zwischen Elektrode und Probe, insbesondere bei Böden, auf.

Vier-Elektroden-Meßanordnungen sind entweder teuer, oder aber der Installationsaufwand ist beträchtlich, da pro Meßposition eine zusätzliche Elektrode eingebracht werden muß, deren Abstand zur zweiten Elektrode nicht genau definiert ist.

Bei flächenhaften und tomographischen Messungen mit sequentieller Stromeinspeisung treten Meßfehler durch Polarisationseffekte an den Elektroden auf, da bei diesen Methoden unmittelbar im Anschluß an eine Stromeinspeisung an diesen, dadurch polarisierten, Elektroden Potentialmessungen durchgeführt werden.

Aufgabe der Erfindung ist es, eine Vorrichtung bereit zu stellen, mit der man gleichzeitig beliebige elektrische Anregungssignale in eine zu untersuchende Probe einspeisen und Potential-Messungen durchführen kann. Mit der Vorrichtung sollen Meßfehler durch Polarisationseffekte ausgeschlossen werden und sie soll zudem einfach aufgebaut sein.

Die Aufgabe wird durch eine Vorrichtung gemäß der Gesamtheit der Merkmale des Hauptanspruchs gelöst. Die Aufgabe wird weiterhin durch eine Vorrichtung gemäß Nebenanspruch gelöst. Vorteilhafte Ausgestaltungen ergeben sich aus den darauf rückbezogenen Patentansprüchen.

Die Aufgabe wird durch eine Vorrichtung zur Einspeisung von beliebigen elektrischen Anregungssignalen und gleichzeitigen Potential-Messung gelöst, die zwei elektrisch voneinander entkoppelte Flächen in einer Elektrodenanordnung umfaßt.
Eine Fläche dient der Einspeisung des beliebigen elektrischen Anregungssignals. Die andere Fläche dient der Messung des Potentials. Da beide Flächen elektrisch voneinander entkoppelt sind, sind die Flächen in einer Elektrodenanordnung realisierbar.

Vorteilhaft ist die Oberfläche der Fläche zur Messung des Potentials kleiner, insbesondere um einen Faktor von mindestens 10 kleiner als die Oberfläche der Fläche zur Einspeisung beliebiger elektrischer Anregungssignale. Dadurch werden die Übergangswiderstände an der Grenzfläche zur Probe klein gehalten und die Messung des Potentials kann an exakt lokalisierten Stellen der zu untersuchenden Probe erfolgen.

Die Vorrichtung kann eine Spitze als Elektrode zur Potentialmessung umfassen. Spitzen lassen sich leicht in eine zu untersuchende Probe einführen. Die Vorrichtung kann dann einen Mantel zur Einspeisung des elektrischen Anregungssignals in die Probe aufweisen.

Vorteilhaft ist die Vorrichtung dann in Form eines Elektrodenspießes mit elektrisch entkoppelten Elektrodenflächen zur Einspeisung der elektrischen Anregungssignale und der Messung des Potentials gelöst. Dabei dient als Elektrode zur Messung des Potentials die Spitze des Elektrodenspießes. Die Spitze dient somit der Ankopplung an die Probe zwecks Potentialmessung.

Der Mantel des Elektrodenspießes dient als Elektrode zur Einspeisung der elektrischen Anregungssignale. Je nach Eindringtiefe des Elektrodenspießes in die Probe existiert somit eine variable Ankopplungsfläche zur Einspeisung des Wechselstroms in die Probe.
Mantel und Spitze des Elektrodenspießes stellen somit die elektrisch entkoppelten Flächen zur Einspeisung des elektrischen Signals und zur Messung des Potentials dar.

Vorteilhaft ist die Vorrichtung einfach aufgebaut. Meßfehler durch Polarisationseffekte sind ausgeschlossen. Zur Installation solcher Elektrodenspieße benötigt man gegenüber der Installation von Elektroden aus dem Stand der Technik nur noch die Hälfte der Zeit. Die Untersuchung einer gegebenen Probenfläche bzw. Probeninhalts wird dadurch beschleunigt, insbesondere bei der Untersuchung komplexer Proben, wie Böden, bei denen mit vielen Elektroden gleichzeitig gearbeitet werden muß.

Die Messung wird durch Einspeisung elektrischer Anregungssignale in die Probe durchgeführt. Mit elektrischen Anregungssignalen sind insbesondere ein konstanter Wechselstrom oder Gleichstrom gemeint, sowie Wechsel- oder Gleichspannung mit variabler Stromstärke, solange die Einspeisung über den Elektrodenspieß-Mantel erfolgt. Beliebiges Signal bedeutet, daß jedwede beliebige Funktionen von der Zeit, z. B. sinus-rechteckige Signale usw. eingespeist werden können. Dadurch wird vorteilhaft z. B. ein Strom über eine relativ große Fläche eingespeist, was den Übergangswiderstand minimiert.

Der Elektrodenspieß weist vorteilhaft eine Spitze zur Messung des Potentials aus Vollmetall auf.
Dadurch ist die Voraussetzung zur Messung des Potentials gegeben. Die Spitze kann auch in dichte Proben, beispielsweise Böden, Aquifere und so weiter eingebracht werden.

Als Elektrode zur Einspeisung der elektrischen Anregungssignale kann ein metallischer, rohrförmiger Mantel dienen. Er ist dazu geeignet die elektrischen Anregungssignale in die zu untersuchende Probe einzuspeisen.

Der Elektrodenspieß-Mantel besteht dann aus einem Metallrohr.

Die Elektrodenspieß-Spitze kann aus edlerem Metall bestehen als der rohrförmige Elektrodenspieß-Mantel.
Im Falle der Einspeisung eines nicht konstanten elektrischen Anregungssignals wird die entstehende Phasenverschiebung zwischen dem elektrischen Signal und dem gemessenen Potential an der Grenzfläche zwischen der Elektrode zur Messung des Potentials und der Probe minimal.

Elektrodenspieß-Mantel und Elektrodenspieß-Spitze sind elektrisch durch isolierendes Material voneinander getrennt. Das isolierende Material kann ringförmig zwischen Elektrodenspieß-Spitze und Elektrodenspieß-Mantel angeordnet sein und trennt diese beiden Teile des Elektrodenspießes elektrisch voneinander.

Zur Ankopplung der Elektrodenspieß-Spitze als Potentialelektrode an weitere Auswerteeinheiten, beispielsweise zur Leitfähigkeitsbestimmung, kann eine flexible Meßleitung zur Übertragung des gemessenen Potentials dienen. Die Meßleitung kann im Innern des Elektrodenspießes angeordnet sein und in einen Koaxialstecker münden. Der Koaxialstecker steht elektrisch wiederum mit weiteren Auswerteeinheiten in Verbindung.

Zur Ankopplung der Potentialelektrode an weitere Meßgeräte, beispielsweise zur Leitfähigkeitsbestimmung, kann auch ein Vollmetallrohr, das an einem Ende die Elektrodenspieß-Spitze trägt, angeordnet sein. Das Vollmetallrohr erhöht die Stabilität der Elektrode zusätzlich.

Eine solche Elektrode ist besonders dazu geeignet in dichte Proben, wie Böden eingebracht zu werden. Bei einer solchen Ausgestaltung ohne flexible Meßleitung weist das Vollmetallrohr einen Anschluß zur Übertragung des gemessenen Potentials in einen Flachbandstecker auf, der wiederum mit weiteren Auswerteeinheiten in Verbindung steht. Über den Flachbandstecker wird einerseits die Übertragung von z. B. einem Wechselstrom auf den Elektrodenspieß-Mantel bewerkstelligt. Andererseits dient er dazu, das an der Spitze gemessene Potential an weitere Auswerteeinheiten zu übertragen.

Alle Isolatoren können besonders vorteilhaft aus mechanisch stabilen, mindestens schlagfesten Kunststoffen bestehen.

Die Elektrode weist vorteilhaft eine Handgriff- oder eine Schlagfläche auf.
Dadurch läßt sich die Elektrode auch in dichte Proben leicht einbringen.

Im weiteren wird die Erfindung an Hand einiger Ausführungsbeispiele und der beigefügten Figuren näher beschrieben.

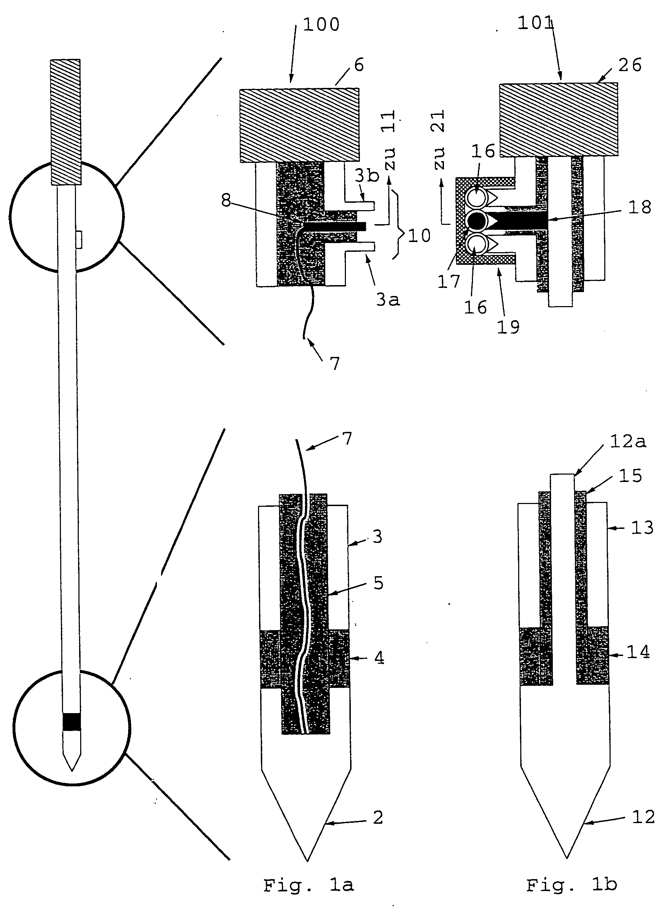

Figuren 1a und b zeigen erfindungsgemäße Vorrichtungen in Form eines Elektrodenspießes 100, 101.
In Figuren 1a und 1b bestehen die Elektrodenspieße 100, 101 aus einer Vollmetallspitze 2, 12, die als Elektrode zur Messung des Potentials dient. Als Elektrode zur Einspeisung des Wechselstroms dient ein Elektrodenspieß-Mantel 3, 13. Elektrodenspieß-Mantel 3, 13 und Vollmetallspitze 2, 12 sind durch isolierendes Material 4, 14 voneinander getrennt. Der Isolator 4, 14 ist ringförmig zwischen Elektrodenspieß-Mantel 3, 13 und Elektrodenspieß-Spitze 2, 12 angeordnet. Ein weiterer Isolator 5, 15 ist im Elektrodenspieß-Mantel 3, 13 angeordnet und trennt Mittel zur Übertragung des in der Spitze 2, 12 gemessenen Potentials von stromführenden Teilen des Elektrodenspießes.

In Figur 1a wird die Übertragung des gemessenen Potentials von der Spitze 2 an eine Auswerteeinheit 11 mittels einer flexiblen Meßleitung 7 bewerkstelligt. Der verbleibende Hohlraum im Elektrodenspieß-Mantel 3 ist zur Verbesserung der mechanischen Stabilität mit Isolator 5 aufgefüllt. Isolator 5 trennt somit die Meßleitung 7 vom stromführenden Elektrodenspieß-Mantel 3. Die flexible Meßleitung 7 mündet am oberen Ende des Elektrodenspießes 1 in einen Anschluß 8 für den Innenleiter einer Koaxialverbindung. Die mit Bezugszeichen 3a und 3b versehenen Verbindungen stellen den Anschluß für den Außenleiter der Koaxialverbindung dar, über die das elektrische Anregungssignal in die Probe eingespeist wird.
Elektrodenspieß-Mantel 3a, 3b sowie Isolator 5 und der Anschluß 8 der flexiblen Meßleitung 7 bilden im oberen Teil der Vorrichtung somit einen Koaxialstecker 10. Dieser verbindet die zwei Elektroden im Elektrodenspieß mit einer nicht dargestellten Steuer- und Auswerteeinheit 11.

Der Elektrodenspieß 100, 101 kann somit beispielsweise zur Messung der Leitfähigkeit eingesetzt werden. Hierzu werden mindestens zwei Elektrodenspieße 1a, 1b in eine Probe eingeführt.

Die Steuer- und Auswerteeinheit 11, 21 umfaßt beispielsweise eine Stromquelle, Meßinstrumente zur Stromstärke und Spannungsmessung, eine programmierbare Steuerung zur sequentiellen Stromeinspeisung an verschiedenen Elektrodenspießen und eine Ausgabeeinheit. Die Elektrode kann weiterhin beispielsweise an einen Mikroprozessor zur Umwandlung analoger in digitale Signale, zur Speicherung, weiteren Umrechnung und/oder Ausgabe der Meßwerte angeschlossen sein. Darüber wird der elektrische Widerstand bzw. als reziproker Wert die Leitfähigkeit der Probe berechnet.

In Figur 1b erhält der Elektrodenspieß seine Stabilität durch eine massive Ausführung des Zentrums des Elektrodenspießes in Form eines Vollmetallrohrs 12a.
Ein Rohr aus isolierendem Material 15 trennt gemeinsam mit Isolierring 14 den Elektrodenspieß-Mantel 13 als Elektrode zur Einspeisung des elektrischen Anregungssignals von dem massiven Vollmetallrohr 12a mit Vollmetallspitze 12 als Elektrode zur Messung des Potentials. Vorteilhaft wird die mechanische Kraft zur Installation der Elektrode in die Probe direkt auf die Spitze 12 übertragen.

In Figur 1b ist zudem ein verpolungssicherer Flachbandstecker 19 gezeigt. Eine Verbindung existiert am oberen Ende des Vollmetallrohrs 12a zum Flachbandstecker 19 über den Anschluß 18. Auch bei dieser Ausführung sind somit spannungs- und stromführende Teile des Elektrodenspießes 1b durch Isolatoren 14, 15 getrennt. Zwei Zuleitungen 16 für das elektrische Anregungssignal sowie die Meßleitung zur Potential-Messung 17 sind ebenfalls dargestellt.

Der Flachbandstecker weist vorteilhaft die Eigenschaft der Verpolungssicherheit auf und ist somit für diese Anwendung, speziell im Feld, besonders geeignet. Der Elektroden-Spieß aus Fig. 1b kann selbstverständlich auch mit einem Koaxialstecker versehen sein.

Die Elektrodenspieße 100, 101 weisen vorteilhaft eine Handgriff- bzw. Schlagfläche 6, 26 auf.
Dadurch läßt sich die Elektrode auch in dichte Proben leicht einbringen.

Ein solcher Elektrodenspieß ist insbesondere in einer Vorrichtung zur schnellen tomographischen Messung der Leitfähigkeitsverteilung in komplexen Proben verwendbar, in der Mittel zur gleichzeitigen Einspeisung der elektrischen Anregungssignale in die Probe vorliegen. Diese Mittel erlauben zusätzlich eine Zuordnung gemessener Potentialdifferenzen in der Probe anteilmäßig zu den eingespeisten elektrischen Anregungssignalen. Als Mittel kommen z. B. eine Steuer- und Recheneinheit in Frage, die die elektrischen Signale in orthogonale elektrische Signale umwandelt und somit eine Zuordnung der gemessenen Potentialdifferenz in der Probe anteilmäßig zu den eingespeisten elektrischen Signalen ermöglicht.

Bei Verwendung nach der Vier-Elektroden-Anordnung erreicht man einen deutlichen Zeitgewinn bei der Auslage der Elektroden, da durch Verwendung von Elektrodenspießen nur die Hälfte der Elektroden gesteckt werden muss.

Weiterhin ist der Abstand zwischen der Einspeisung des elektrischen Anregungssignals und der Potentialmessung an der erfindungsgemäßen Vorrichtung, insbesondere bei einem Elektrodenspieß konstruktionsbedingt konstant. Für die geophysikalische Anwendung ist dieser Abstand darüber hinaus vernachlässigbar gering.

Bei allen Messungen, die an einer Elektrode gleichzeitig Strom einspeisen und das Potential messen, werden die Meßfehler aufgrund der Kontaktwiderstände aufgehoben, da durch die Elektrodenspießspitze, die als Potentialmeßelektrode dient, kein Strom fließt und somit an diesem Kontaktwiderstand kein Spannungsabfall auftritt.

Grundsätzlich ergibt sich für alle Meßmethoden der Vorteil, daß die durch Polarisationseffekte bedingten Meßfehler wegen der strikten Trennung von Strom- und Potentialelektrode nicht auftreten können.

Ein weiterer Vorteil ist die kostengünstigere Herstellung von Spezialelektroden zur Messung der induzierten Polarisation der Proben. Dort wird die Phasenverschiebung eines eingespeisten Wechselstromes gemessen, wobei die Phasenverschiebung an der Grenzfläche zwischen der Elektrode zur Messung des Potentials und der Probe minimal sein soll, was durch die Verwendung von Edelmetallen als Material für die Elektrode zur Messung des Potentials erreicht werden kann. Für die Elektrode zur Einspeisung des Wechselstroms ist dieser Aufwand nicht nötig, da deren Einfluß auf die Phase des Wechselstromes irrelevant ist. Durch die Trennung und Aufteilung der Funktion des Einspeisens von Strom und zur Messung des Potentials auf zwei Elektroden, macht die Oberfläche der Potentialelektrode nur einen kleinen Teil der Gesamtoberfläche aus. Daher ist es in der Herstellung kostengünstig möglich, für die Potentialelektrode ein edleres Metall zu verwenden, als für die Stromeinspeisungselektrode.

Es ist grundsätzlich möglich, die erfindungsgemäße Vorrichtung und insbesondere den Elektrodenspieß auf folgenden Gebieten einzusetzen:
- Ermittlung der Konzentration gelöster Substanzen in Böden und Grundwasserleitern aus der Leitfähigkeitsverteilung.
- Beobachtung chemischer Prozesse durch Messung der zeitlichen und räumlichen Variationen der Leitfähigkeitsverteilung (z. B. Fällungsreaktionen).
- Aufspüren und Untersuchen von elektrischen Anomalien (metallische Körper, Erze, Leitungen, Blindgänger, archäologische Objekte etc.).
- Erkundung und Monitoring bevorzugter Fließ- und Transportwege in porösen Medien (z.B. Böden, Aquifere. Kluftgesteine, Laboranordnungen).
- Verlagerung von Agrochemikalien.
- Unterstützung des Katastrophenmanagements bei Industrieunfällen.
- Gefährdungsabschätzung für Industriestandorte.
- Monitoring bei Labor- und Freilandversuchsfeldern zur Untersuchung von Transportvorgängen in porösen Medien.
- Bestimmung von Partikel- oder kolloidalen Transport.
- Aufspüren und Untersuchen von besonders schlecht leitenden Flüssigkeiten in Böden und Grundwasserleitern (durch die Verdrängung des Wasserfilms auf den Körnern), etwa:
   - Schadstofffahnen (Öle, PAKs)
   - Sanierung von Böden
   - Monitoring von Deponien auf Undichtigkeiten
   - Monitoring von Deichen auf Durchweichung.
   - Erkundung von wassertragenden oder wasserführenden Schichten in ariden oder semiariden Gebieten.
   - Erkundung von Salz-Süßwasserfronten im Untergrund, in Flußsystemen oder in Agroökosystemen.

Insbesondere der erfindungsgemäße Elektrodenspieß kann durch geschickte Auswahl der Abmessungen und der verwendeten Materialien auch in der Medizin (Gewebeproben) oder in der Lebensmitteltechnologie eingesetzt werden. Auch flüssige Proben können charakterisiert werden.

Je nach zu untersuchender Probe kann die erfindungsgemäße Vorrichtung weitere beliebige Formen aufweisen, z. B. auch flächig ausgestaltet sein um einen besseren Stoffschluß zur Probe zu erzielen.

## Patentansprüche

1. Vorrichtung zur Einspeisung von elektrischen Anregungssignalen und gleichzeitigen Potential-Messung in Proben,
**gekennzeichnet durch**
zwei elektrisch entkoppelte Flächen in einer Elektrodenanordnung, wobei eine Fläche zur Einspeisung beliebiger elektrischer Anregungssignale und die andere Fläche zur Messung des Potentials dient.

2. Vorrichtung nach Anspruch 1,
**dadurch gekennzeichnet, daß**
die Oberfläche der Fläche zur Messung des Potentials kleiner ist, insbesondere um mindestens einen Faktor 10 kleiner ist, als die Oberfläche der Fläche zur Einspeisung beliebiger elektrischer Anregungssignale.

3. Vorrichtung nach einem der vorhergehenden Ansprüche,
**dadurch gekennzeichnet, daß**
die zwei elektrisch entkoppelten Flächen in einer Elektrodenanordnung durch einen ringförmigen Isolator voneinander getrennt sind.

4. Vorrichtung nach einem der vorhergehenden Ansprüche,
umfassend eine Spitze als Elektrode zur Potentialmessung.

5. Vorrichtung nach einem der vorhergehenden Ansprüche,
**gekennzeichnet durch**
einen Mantel zur Einspeisung des elektrischen Anregungssignals in die Probe.

6. Vorrichtung nach einem der vorhergehenden Ansprüche,
**gekennzeichnet durch**
einen Elektrodenspieß (100, 101).

7. Vorrichtung nach Anspruch 6,
**gekennzeichnet durch**
eine Elektrodenspieß-Spitze (2, 12) aus Vollmetall.

8. Vorrichtung nach Anspruch 6 oder 7,
**gekennzeichnet durch**
ein Metallrohr als Elektrodenspieß-Mantel (3, 13).

9. Vorrichtung nach einem der Ansprüche 6 bis 8,
**dadurch gekennzeichnet, daß**
die Elektrodenspieß-Spitze aus einem edleren Metall als der Elektrodenspieß-Mantel besteht.

10. Vorrichtung nach einem der Ansprüche 6 bis 9,
**dadurch gekennzeichnet, daß**
Elektrodenspieß-Mantel (3, 13) und Elektrodenspieß-Spitze (2, 12) durch einen ringförmigen Isolator (4, 14) elektrisch voneinander getrennt sind.

11. Vorrichtung nach einem der Ansprüche 6 bis 10,
**gekennzeichnet durch**
eine flexible Meßleitung (7) zur Übertragung des gemessenen Potentials von der Elektrodenspieß-Spitze (2, 12) an weitere Auswerteeinheiten (11, 21).

12. Vorrichtung nach einem der Ansprüche 6 bis 11,
**gekennzeichnet durch**
einen Isolationskörper (5) der die flexible Meßleitung (7) elektrisch von dem Elektrodenspieß-Mantel (3) trennt.

13. Vorrichtung nach einem der Ansprüche 6 bis 12,
**gekennzeichnet durch**
ein Vollmetallrohr (12a) zur Übertragung des gemessenen Potentials von der Elektrodenspieß-Spitze (12) an weitere Auswerteeinheiten (21).

14. Vorrichtung nach Anspruch 13,
**gekennzeichnet durch**
einen Isolationsrohr (15) zur elektrischen Isolierung des Vollmetallrohrs (12a) von dem Elektrodenspieß-Mantel (13).

15. Vorrichtung zur Messung der Leitfähigkeit, umfassend mindestens zwei Vorrichtungen nach einem der vorhergehenden Ansprüche.

16. Vorrichtung zur Messung der dreidimensionalen, tomographischen Leitfähigkeitsverteilung einer Probe, umfassend eine Vielzahl von Elektrodenspießen (100, 101) nach einem der Ansprüche 5 bis 14.
